# EUROPEAN PATENT APPLICATION

(11) **EP 0 684 224 A1**
(43) Date of publication of application: **29.11.1995**
(21) Application number: 95103315.8
(22) Date of filing: 08.03.1995
(51) Int. Cl.: C07C 217/16, C07C 213/08, C07C 255/13

(54) **A process for the preparation of 2-(4-N-methylaminobutoxy)diphenylmethane**

(30) Priority: 24.03.1994 IT MI940563
(71) Applicant: PROCOS S.p.A., I-28062 Cameri (Novara) (IT)
(72) Inventor: Lavacchielli, Augusto, I-28062 Cameri (Novara) (IT); Fornaroli, Mirco, I-28062 Cameri (Novara) (IT); Velardi, Francesco, I-28062 Cameri (Novara) (IT)
(74) Representative: Minoja, Fabrizio

(57) **Abstract**

A process for the preparation of 2-(4-N-methylaminobutoxy)diphenylmethane of formula (I) 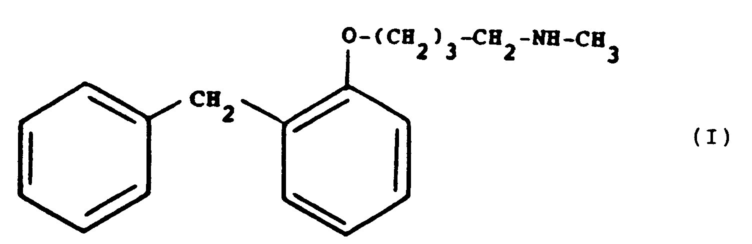
and the pharmaceutically acceptable acid salts thereof, starting from 2-(3-cyanopropoxy)diphenylmethane.

## Description

The present invention relates to a process for the preparation of 2-(4-N-methylaminobutoxy)diphenylmethane and the pharmaceutically acceptable acid salts thereof.

Compound 2-(4-N-methylaminobutoxy)diphenylmethane of formula (I) 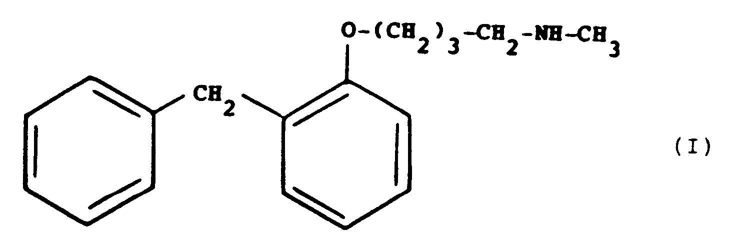
also known as Bifemelane, has pharmacological activity, particularly it is used as an antidepressant.

Compound of formula (I) belongs to a class of 2-omega-aminoalkoxydiphenylmethanes described in US 4.091.114, granted on May 23, 1978, in the name of Mitsubishi Chemical Industries Ltd. Subsequent references disclose the above cited compound, for example EP 0 103 897, JP 1.022.847 and Kikumoto et al., Journal Medicinal Chemistry, 1981, 24, 145-148.

The US-Patent discloses a process for the preparation of Bifemelane, which comprises reacting 2-(4-halogen)butoxydiphenylmethane with an equimolar amount of methylamine.

However, in order to speed up the reaction, an amine molar excess up to 100 times the substrate is provided for. The resulting compound can be salified with a pharmaceutically acceptable acid.

2-(4-Bromobutoxy)diphenylmethane is in its turn prepared by reacting 2-hydroxydiphenylmethane with 1,4-dibromobutane.

The latter is used in a considerable excess to prevent the dimer from forming, and a major part of this bromo-derivative is destroyed during the reaction, thereby it can be recycled only partially; moreover, the obtained intermediate product is not pure, but is obtained in a variable purity (80-90%) together with other by-products.

The subsequent N-alkylation with methylamine yields a product which is difficult to purify, due to the presence of di- and tri-derivatives and methylamine ammonium salts.

Now it has been found, and it is the object of the present invention, a process for the preparation of 2-(4-N-methylaminobutoxy)diphenylmethane and the pharmaceutically acceptable acid salts thereof, which avoids the use of the above mentioned reagents, i.e. halogen derivatives and alkylamine, and allows to obtain purer products, thus overcoming the problems related to the presence of by-products.

According to the present invention, the process for the preparation of 2-(4-N-methylaminobutoxy)diphenylmethane comprises the following steps:
a) reaction of 2-(3-cyanopropoxy)diphenylmethane of formula (II) 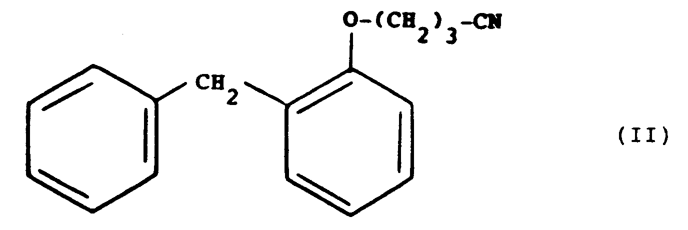 with a suitable reducing agent to give 2-(4-aminobutoxy)diphenylmethane of formula (III) 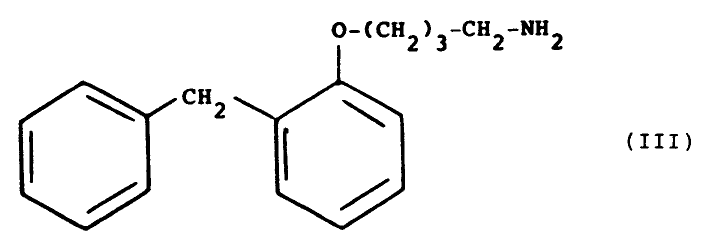
b) treatment of compound (III) with an amino nitrogen methylation agent;
c) optional salification with a pharmaceutically acceptable acid.

In a first preferred embodiment, the starting compound (II) is subjected to catalytic hydrogenation. For example, the hydrogenation reaction is carried out at a temperature from 40 to 100°C under a pressure from 5 to 50 at. (490.3 kPa - 4903 kPa) with a Nickel Raney catalyst in the presence of ammonia. The reaction time varies from 2 to 8 hours depending on the temperature. Aliphatic alcohols having 1 to 5 carbon atoms, preferably methanol, ethanol and isopropanol, can be used as solvents.

In the subsequent step, according to a first preferred embodiment of the present invention, the amine obtained from the nitrile reduction is methylated with a suitable reagent, such as dimethylsulfate or a methyl halide. In a second preferred embodiment, in order to prevent the formation of undesired by-products, such as tertiary amines and ammonium salts, amine (III) is treated first with a suitable aldehyde, preferably benzaldehyde to give the relative Schiff's base, and subsequently with the methylation agent.

The reaction of the primary amine (III) with aldehyde is carried out at a temperature ranging from 80 to 150°C, optionally in the presence of an inert solvent, for example an aromatic hydrocarbon, such as benzene, toluene o xylene, until the water formed during the reaction is removed. After that, the methylation agent is added, optionally dissolved in the same reaction solvent, keeping reflux for 0.5-4 hours. Subsequently, the solvent is removed, for example by distillation, water is added to hydrolyze the Schiff's base and reflux is continued for 5 minutes to 3 hours, depending on the methylation agent used. The aldehyde is either distilled off in a vapour stream, or extracted with a suitable solvent. Then the final product is freed with a strong base, such as an alkali metal hydroxide and extracted with a suitable solvent, for example an ether or an aromatic hydrocarbon, preferably diethyl ether, benzene, toluene, xylene.

The reaction intermediates and the final product (I) can be recovered by conventional processes known to those skilled in the art. In a preferred embodiment of the invention, the products are recovered by distillation under vacuum. However, other methods can also be used, for example the various known chromatography techniques or crystallization.

The salification of compound of formula (I) is also performed conventionally.

Examples of pharmaceutically acceptable salts are those obtained with hydrochloric, hydrobromic, sulfuric, phosphoric, nitric, acetic, adipic, propionic, tartaric, maleic, citric, benzoic, methanesulfonic acids and the like.

The compound 2-(3-cyanopropoxy)diphenylmethane of formula (II) is novel and therefore is another object of the present invention, together with the process for the preparation thereof, described in the following.

The preparation of 2-(3-cyanopropoxy)diphenylmethane comprises the reaction of 2-hydroxydiphenylmethane of formula (IV) 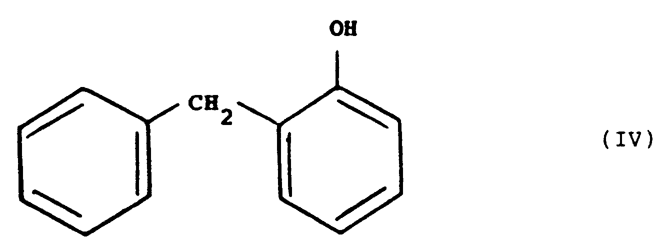
with a 4-(halogen)butyronitrile of formula (V)

X-(CH₂)₃-CN (V)

wherein X is a halogen atom, such as chlorine, bromine, iodine, preferably chlorine.

Compounds (IV) and (V) are commercially available or they can be prepared according to what described in literature.

The reaction takes place in dimethylformamide or in polyethylene glycol in the presence of an inorganic base, for example an alkali metal carbonate or bicarbonate, such as sodium or potassium, or an organic base, for example a tertiary amine, such as triethylamine and the like, in a ratio of compound (IV) to base ranging from 1:1 to 1:4; the molar ratio of halonitrile to compound (IV) ranging from 1.1 to 1.4; the temperature of reaction varying from 70 to 160°C for times between 2 and 18 hours.

Alternatively, the reaction can be carried out in a phase-transfer catalysis, according to conventional procedures.

Compared with the prior art, the process according to the present invention gives remarkable advantages as far as the higher quality of the final product and the possibility of more efficiently checking and purifing the intermediates are concerned, as well as from the cost-savings point of view, since yields are, on average, higher, and, last but not least, in view of environmental matters, due to the lower presence of by-products to be removed during the production.

The following examples further illustrate the invention.

The abbreviations used in the examples have the following meanings:
- DMF: = dimethylformamide
- G.C.: = gaschromatography;
- m.p.: = melting point

### Example 1

100 g (0.54 moles) of 2-benzylphenol are dissolved in 400 ml of DMF and 200 g (1.45 moles) of potassium carbonate and then 64 g (0.61 moles) of 4-chlorobutyronitrile are added thereto. The mixture is heated to 140°C for 5 hours. When the reaction is over, DMF is distilled under vacuum and toluene (250 ml) and water (250 ml) are added. The phases are separated. The solvent is evaporated from the toluene extract with suction (20-30 torrs) to obtain a residual oil which is distilled under a vacuum of 5 torrs at a temperature of 170-180°C in the head. 116.6 g are obtained, which crystallize with a 98.2% gaschromatography titre. Yield: 86%.

### Example 2

100 g (0.54 moles) of 2-benzylphenol are dissolved in 200 ml of polyethylene glycol-200 and 200 g (1.45 moles) of potassium carbonate and then 64 g (0.61 moles) of 4-chlorobutyronitrile are added thereto. The reaction mixture is heated to 140°C for 5 hours. When the reaction is over, toluene (250 ml) and water (500 ml) are added. The phases are separated. The solvent is evaporated from the toluene extract with suction (20-30 torrs) to obtain a residual oil which is distilled under a vacuum of 5 torrs, at a temperature of 170-180°C at the head. 130 g are obtained, which crystallize, m.p. = 50-51.2°C, with a 98.5% gaschromatography titre. Yield: 94%.

### Example 3

300 g (1.19 moles) of 2-(3-cyanopropoxy)diphenylmethane, 1400 ml of methanol, 17% ammonia in methanol (300 ml) and 20 g of nickel Raney are placed into an autoclave. The mixture is hydrogenated at 40°C until complete absorption, filtered and the solvent is evaporated off under vacuum, then the residue is distilled at 170-180°C under 3 torrs. A thick liquid is obtained, 266.5 g; 99% gaschromatography titre. Yield: 87,7%.

### Example 4

60 g (0.235 moles) of 2-(4-aminobutoxy)diphenylmethane, 120 ml of toluene and 34 g (0.32 moles) of benzaldehyde are placed into a 1 l round-bottomed flask. The mixture is heated to 95-100°C for 30 minutes, then toluene is distilled off and fresh toluene (80 ml) is added, cooled to 40°C. A mixture of dimethylsulfate (40 g, 0.32 moles) and toluene (120 ml) is added thereto. After one hour under reflux, water (180 ml) is added drop by drop and reflux is continued for a further 20 minutes. Toluene is distilled off, then the developed benzaldehyde is distilled in a vapour stream. After cooling, the base is freed with 30% NaOH, extracting it with toluene. Toluene is removed and 2-(4-methylaminobutoxy)diphenylmethane is distilled under a 3 torrs vacuum, at a temperature of 150-160°C. 47.1 g are obtained, with a 98.9% G.C. titre.

### Example 5

30 g of distilled 2-(4-methylaminobutoxy)diphenylmethane are dissolved in acetone (300 ml) and gaseous hydrochloric acid is bubbled until the mixture is acidic. 2-(4-Methylaminobutoxy)diphenylmethane hydrochloride precipitates, which is filtered, washed on the filter with acetone and dried in oven at 100°C. 30.2 g of a product are obtained, with 99.6% chemical titre, m.p. = 119-120°C and 99.7% G.C. titre.

By way of comparison the following table reports yields and melting points of the 2-(4-N-methylaminobutoxy)diphenylmethane hydrochloride obtained according to the present invention (A) and according to Journal of Medicinal Chemistry, 1981, Vol. 24, No. 2 (B) and Example N. 4 of U.S. 4.091.114 (C).

**Table**

| Melting point and yield of 2-(4-N-methylaminobutoxy)diphenylmethane hydrochloride. | | |
|---|---|---|
| Source | Yield % | Melting point |
| A | 88.78 | 119-120 |
| B | 73.0 | 117-121 |
| C | n.d. | 114-119 |
| n.d.= not determined. | | |

As is clearly shown from the above table, the process of the present invention yields a purer product in higher yields.

## Claims

1. A process for the preparation of 2-(4-N-methylaminobutoxy)diphenylmethane of formula (I) 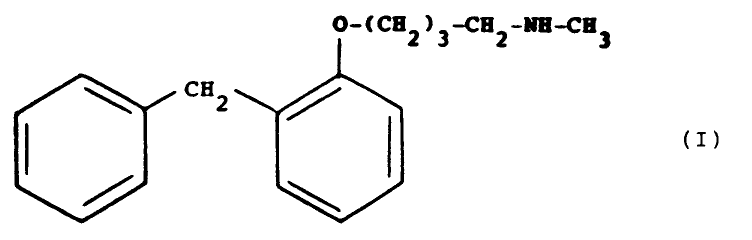 and the pharmaceutically acceptable acid salts thereof, which comprises the following steps:
a) reaction of 2-(3-cyanopropoxy)diphenylmethane of formula (II) 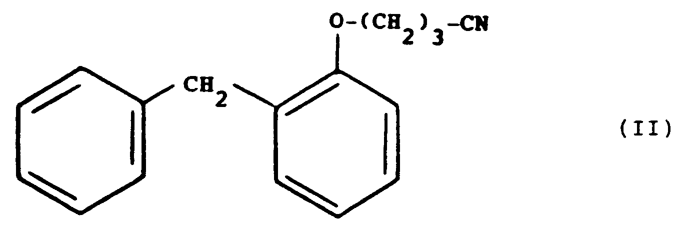 with a suitable reducing agent to give 2-(4-aminobutoxy)diphenylmethane of formula (III) 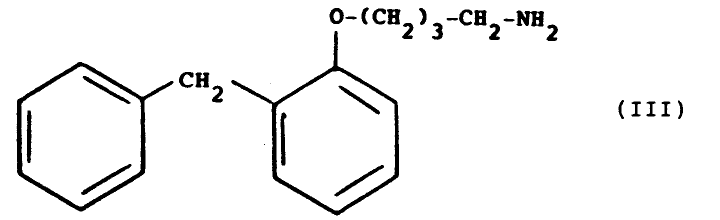
b) treatment of compound (III) with an amino nitrogen methylation agent;
c) optional salification with a pharmaceutically acceptable acid.

2. A process according to claim 1, in which the reaction of step a) is carried out by means of catalytic hydrogenation, using preferably Nickel Raney as catalyst.

3. A process according to claim 1 or 2, in which the reaction in step b) is carried out with dimethylsulfate as methylation agent.

4. A process according to any one of claims 1 to 3, in which the reaction in step b) is carried out in the presence of a solvent, preferably an aromatic hydrocarbon.

5. A process according to any one of claims 1 to 4, in which the reaction in step b) is carried out in the presence of an aldehyde.

6. A process according to any one of claims 1 to 5, in which said aldehyde is benzaldehyde.

7. A process according to any one of claims 1 to 6, in which step b) comprises:
b.1) reaction of said compound of formula (III) with said aldehyde, preferably benzaldehyde, to give the corresponding Schiff's base, optionally in the presence of a solvent, until the water formed in the reaction is removed;
b.2) addition of the methylation agent, optionally dissolved in the reaction solvent;
b.3) elimination of the reaction solvent;
b.4) hydrolysis of said Schiff's base and elimination of said aldehyde;
b.5) treatment of the product with a strong base.

8. A process according to any one of claims 1 to 7, in which, in step c), said acid is hydrochloric acid.

9. 2-(3-Cyanopropoxy)diphenylmethane of formula (II)

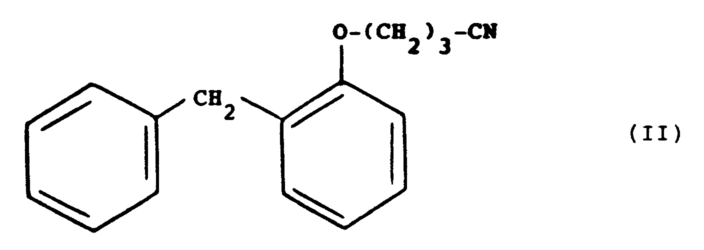

10. A process for the preparation of the compound of claim 9, which comprises the reaction of 2-hydroxydiphenylmethane of formula (IV) 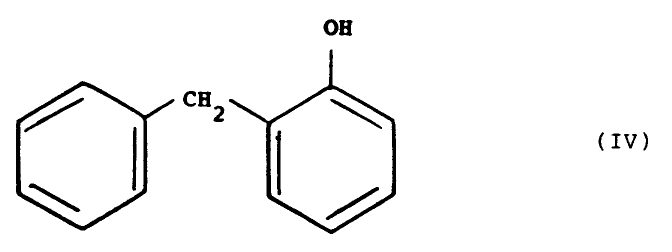 with a 4-(halogen)butyronitrile of formula (V)
X-(CH₂)₃-CN (V)
wherein X is a halogen atom, such as chlorine, bromine, iodine.

11. A process according to claim 10 in which the reaction of compound (IV) with compound (V) is carried out in a solvent selected from dimethylformamide and polyethylene glycol.

12. A process according to claim 10 or 11, in which the reaction of compound (IV) with compound (V) is carried out in the presence of an organic or inorganic base and the molar of compound (IV) to compound (V) ranges from 1.1 to 1.4.

13. A process according to claims 10-12, in which the reaction is carried out in a phase-transfer catalysis.
